Europäisches Patentamt

European Patent Office

Office européen des brevets

(19)

(11) Veröffentlichungsnummer: **0 118 114**
**A1**

(12) ## EUROPÄISCHE PATENTANMELDUNG

(21) Anmeldenummer: 84102207.2

(22) Anmeldetag: 02.03.84

(51) Int. Cl.³: **A 61 M 1/03**
**A 61 J 1/00**

(30) Priorität: 03.03.83 DE 8305980 U

(43) Veröffentlichungstag der Anmeldung:
12.09.84 Patentblatt 84/37

(84) Benannte Vertragsstaaten:
AT BE CH DE FR GB IT LI NL

(71) Anmelder: SCHI-WA Arzneimittelwerk GmbH

D-4519 Glandorf(DE)

(72) Erfinder:
Der Erfinder hat auf seine Nennung verzichtet

(74) Vertreter: Sand, Jakob, Dr. et al,
c/o BASF Aktiengesellschaft Patentabteilung ZNP - C6
Carl-Bosch-Strasse 38
D-6700 Ludwigshafen(DE)

(54) Doppelkammerbeutel für Stoffaustauschapparate zur extrakorporalen Blutreinigung.

(57) Doppelkammerbeutel für Stoffaustauschapparate zur extrakorporalen Blutreinigung, bestehend aus einer äußeren, reckfesten Beutelhülle (1), die durch eine Trennwand (2) in eine erste Kammer (3) zum Aufnehmen der dem Körperkreislauf entnommenen Flüssigkeit und in eine zweite Kammer (5) für eine durch Verdrängung in den Körperkreislauf zurückzuführende Ersatzflüssigkeit unterteilt ist, wobei die Kammern mit jeweils einem Anschluß (4, 6) versehen sind.

EP 0 118 114 A1

Croydon Printing Company Ltd

Doppelkammerbeutel für Stoffaustauschapparate zur extrakorporalen Blutreinigung

Die Erfindung betrifft einen Doppelkammerbeutel für Stoffaustauschapparate zur extrakorporalen Blutreinigung.

Beim Stoffaustausch zur extrakorporalen Blutreinigung, beispielsweise zur Blutentgiftung durch Behandlungsverfahren wie Hämodialyse, Hämofiltration, Spontanfiltration, Hämodiafiltration, Plasmaseparation, ist die dem Körperkreislauf entnommene Flüssigkeit durch eine sterile Substitutionsflüssigkeit simultan und volumengleich zu ersetzen. Für diese Bilanzierung stehen grundsätzlich zwei Verfahren zur Verfügung:

1. gravimetrische Bilanzierung
2. volumetrische Bilanzierung

Bei der gravimetrischen Methode werden entnommene Flüssigkeit und Substitutionsflüssigkeit ständig gewogen und die Summe ihrer Gewichte gesteuert, indem die von der zulaufenden Flüssigkeit verursachte Abweichung vom bei Behandlungsbeginn registrierten Sollgewicht durch Zurückführen von Substitutionsflüssigkeit in den Körperkreislauf ausgeglichen wird. Hierzu sind Wägevorrichtungen vorgesehen, die über Steuereinrichtungen auf eine Pumpe für die entnommene Flüssigkeit und eine Pumpe für die Substitutionsflüssigkeit einwirken.

Bei der volumetrischen Methode sind anstelle der Wägevorrichtung starre Volumenmeßkammern vorgesehen, durch die eine Volumenportionierung erreicht wird. Das System wird durch eine Pumpe für die entnommene Flüssigkeit angetrieben.

Beide Bilanzierungsprinzipien benötigen einen hohen gerätetechnischen Aufwand, wie eichpflichtige und hochpräzise Wägevorrichtungen, Volumenmeßkammern, spezielle Ventile, Steuer- und Kontrolleinrichtungen. Verbunden mit dem aufwendigen Gerätesystem sind Fehlerquellen, die die Sicherheit beeinträchtigen können.

Angestrebt wird daher eine einfache, möglichst steuerungslose Bilanzierungstechnik, bei der die Flüssigkeitssubstitution über einen längeren Zeitraum sowohl bei geringster Abweichung der Gesamtmenge als auch während der Austauschbehandlung zeit- und volumengleich erfolgen Sp/ro soll. Dabei müssen Sterilität und Zuverlässigkeit der tatsächlich ausgetauschten Flüssigkeitsmengen über den gesamten Einsatzzeitraum sichergestellt sein.

Vorliegender Erfindung liegt daher die Aufgabe zugrunde, eine Einrichtung
für Stoffaustauschapparate zur extrakorporalen Blutreinigung zu entwickeln, bei der die vorstehenden Forderungen erfüllt sind.

Gelöst wird die Aufgabe durch einen Doppelkammerbeutel für Stoffaustauschapparate mit den in den Patentansprüchen beschriebenen Merkmalen.

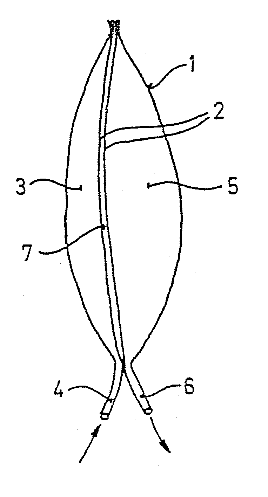

Der erfindungsgemäße Doppelkammerbeutel ist nachfolgend anhand eines in
der Zeichnung schematisch dargestellten Ausführungsbeispiels näher erläutert.

Der als Einmalsystem konzipierte Doppelkammerbeutel besteht aus einer
äußeren Beutelhülle 1, die durch eine flexible Trennwand 2 in zwei
Kammern unterteilt ist. Eine erste Kammer 3 nimmt über einen Anschluß 4
die dem Körperkreislauf durch einen Stoffaustauschapparat entnommene
Flüssigkeit auf, die mit Hilfe einer handelsüblichen Rollenpumpe luftfrei
zugeführt wird. Die zweite Kammer 5 ist von vornherein mit einer sterilen
Ersatzflüssigkeit gefüllt. Diese wird von der in die erste Kammer gepumpten Flüssigkeit durch Verschieben der Trennwand verdrängt und dadurch
über einen Anschluß 6 in einen venösen Zugang zum Körperkreislauf gefördert. Dabei ist der Doppelkammerbeutel ohne hydrostatisches Gefälle zum
Körperkreislauf plaziert.

Für ein konstantes Beutelvolumen und damit eine Verdrängung im Verhältnis
1 : 1 (volumengleich) besteht die äußere Beutelhülle 1 aus reckfestem
Material, beispielsweise Polyterephthalsäure-ester-Verbundfolie. Dabei
muß die Trennwand 2 ohne nennenswerten Bewegungswiderstand sein.

Eine vorteilhafte Ausführungsform des Doppelkammerbeutels besteht darin,
daß die Trennwand doppelt ausgeführt ist. Dadurch entsteht eine dritte
Kammer 7 als Sicherkeitskammer. Diese Kammer kann mit geringen Mengen
eines beliebigen flüssigen oder gasförmigen Stoffes gefüllt sein, der
Indikation oder Schutzfunktion bei einem Leck übernimmt.

Es versteht sich, daß im Rahmen vorliegender Erfindung je nach Anwendungsfall noch weitere Ausführungsformen möglich sind. Beispielsweise können
die beiden Anschlüsse an entgegengesetzten Enden des Beutels angebracht
sein; ferner kann die Doppelkammer durch "Beutel im Beutel" gebildet sein
oder zwei Beutel am Rand zusammengeschweißt sein. Die Anwendung des Doppelkammerbeutels ist auch nicht auf die Blutreinigung beschränkt, sondern kann auch für andere Behandlungen mit ähnlichen Verfahrensvorgängen eingesetzt werden, z.B. zur Spüllösungsverdrängung bei orthopädischen Operationen.

Durch den Doppelkammerbeutel kann nun auf Meß- und Steuereinrichtungen verzichtet werden. Dadurch ist ein Stoffaustausch praktisch an jedem Ort, insbesondere in Intensivstationen oder Operationsräumen ohne das Vorhandensein spezieller Gerätschaften möglich. Dabei kann der Doppelkammerbeutel mit wenigen, dem Pflegepersonal vertrauten Handgriffen wie bei einer üblichen Infusionstherapie mit Pumpenunterstützung angeschlossen werden.

## Patentansprüche

1. Doppelkammerbeutel für Stoffaustauschapparate zur extrakorporalen Blutreinigung, gekennzeichnet durch eine äußere, reckfeste Beutelhülle (1), die durch eine flexible Trennwand (2) in eine erste Kammer (3) zum Aufnehmen der dem Körperkreislauf entnommenen Flüssigkeit und in eine zweite Kammer (5) für eine durch Verdrängung in den Körperkreislauf zurückzuführende Ersatzflüssigkeit unterteilt ist, wobei die Kammern mit jeweils einem Anschluß (4,6) versehen sind.

2. Doppelkammerbeutel nach Anspruch 1, dadurch gekennzeichnet, daß die Trennwand (2) doppelt ausgeführt ist, so daß zwischen den beiden Kammern (3,5) eine dritte Kammer (7) als Sicherheitskammer gebildet ist.

Zeichn.

O.Z. 0816/00008

Europäisches
Patentamt

EUROPÄISCHER RECHERCHENBERICHT

0118114
Nummer der Anmeldung

EP 84 10 2207

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y | DE-A-2 755 882 (STREICHER) <br> * Insgesamt und insbesondere Figur 4; Seite 4, Zeilen 5-12; Seite 6, Zeilen 7-11; Seite 7, Zeilen 6-12, 14-16 * | 1,2 | A 61 M 1/03 <br> A 61 J 1/00 |
| Y | US-A-3 838 794 (COGLEY) <br> * Figur 2; Spalte 2, Zeilen 48-50 * | 1,2 | |
| Y | DE-A-2 006 054 (SCHÖNBACH) <br> * Figur 2; Seite 3, Zeilen 13-20 * | 2 | |
| X | AT-B- 198 424 (SCHÜRER-WALDHEIM) <br> * Insgesamt; insbesondere Abbildung; Seite 1, Zeilen 63-72 * | 1 | RECHERCHIERTE SACHGEBIETE (Int. Cl. ³) |
| E | WO-A-8 302 061 (BAXTER) <br> * Figuren 1,2; Seite 6, Zeile 33 - Seite 7, Zeile 12 * | 1 | A 61 M <br> A 61 J |

Der vorliegende Recherchenbericht wurde für alle Patentansprüche erstellt.

| Recherchenort <br> DEN HAAG | Abschlußdatum der Recherche <br> 21-05-1984 | Prüfer <br> PETZUCH M. |
|---|---|---|